# EUROPÄISCHE PATENTSCHRIFT

(11) **EP 0 686 443 B1**
(45) Veröffentlichungstag und Bekanntmachung des Hinweises auf die Patenterteilung: **10.11.1999**
(21) Anmeldenummer: 95107643.9
(22) Anmeldetag: 19.05.1995
(51) Int. Cl.: B22C 9/06, B22D 13/10, B22D 13/06

(54) **Verfahren zum Herstellen von Gussteilen aus reaktiven Metallen und wiederverwendbare Giessform zur Durchführung des Verfahrens**
Method for the production of castings of reactive metals and reusable mould for carrying it out
Procédé de fabrication de pièces coulées d'un métal réactif et moule réutilisable pour la mise en oeuvre de la méthode

(30) Priorität: 09.06.1994 DE 4420138; 20.02.1995 DE 19505689
(43) Veröffentlichungstag der Anmeldung: 13.12.1995
(73) Patentinhaber: ALD Vacuum Technologies GmbH, 63526 Erlensee (DE)
(72) Erfinder: Choudhury, Alok, Dr., D-66346 Püttlingen (DE); Blum, Matthias, Dr., D-63654 Büdingen (DE); Scholz, Harald, Dipl.-Ing., D-63517 Rodenbach (DE); Jarczyk, Georg, Dipl.-Ing., D-63538 Grosskrotzenburg (DE)
(74) Vertreter: Zapfe, Hans, Dipl.-Ing.

(56) Entgegenhaltungen:
- EP-A- 0 443 544
- PATENT ABSTRACTS OF JAPAN vol. 13 no. 296 (M-846) ,10.Juli 1989 & JP-A-01 087050 (TOOKARO KK) 31.März 1989,
- PATENT ABSTRACTS OF JAPAN vol. 18 no. 62 (M-1553) ,2.Februar 1994 & JP-A-05 285624 (MITSUBISHI MATERIALS CORP) 2.November 1993,

## Beschreibung

Die Erfindung betrifft ein Verfahren zum Herstellen von Gußteilen nach dem Oberbegriff des Patentanspruchs 1, sowie eine Gießform gemäß dem Oberbegriff des Anspruchs 8.

Es besteht ein steigender Bedarf an Bauteilen aus Titan oder aus Legierungen mit einem beträchtlichen Titananteil, da diese Werkstoffe ein geringes spezifisches Gewicht und dennoch eine hohe Festigkeit aufweisen, vorausgesetzt, man beachtet in ausreichendem Umfange die spezifischen Eigenschatten des Titans, zu denen sein hoher Schmelzpunkt und seine Reaktionsfreudigkeit bei hohen Temperaturen gehören. Bei Schmelztemperatur reagiert Titan nicht nur mit reaktionsfähigen Gasen, darunter insbesondere Sauerstoff, sondern auch mit Oxiden und nahezu allen Keramiken, da diese üblicherweise zumindest überwiegend aus oxidischen Verbindungen bestehen. Durch die größere Affinität des Titans zu Sauerstoff wird den Oxiden Sauerstoff entzogen und führt zur Bildung von Titanoxiden. Einige Werkstoffe, die sich für bestimmte Einsatzgebiete hervorragend bewährt haben, werden nachstehend beispielhaft aufgeführt:
Reintitan
Ti 6 Al 4 V
Ti 6 Al 2 Sn 4 Zr 2 Mo
Ti 5 Al 2,5 Sn
Ti 15 V 3 Al 3 Cr 3 Sn
Ti Al 5 Fe 2,5
50 Ti 46 Al 2 Cr 2 Nb
Titanaluminide.

Besonders zu erwähnen ist der Einsatz von Titanaluminiden, z.B. TiAl als Werkstoff für zahlreiche Bauteile. Aufgrund ihrer geringen Dichte, relativ hohen Wärmefestigkeit und Korrosionsbeständigkeit gelten die Titanaluminide als optimaler Werkstoff für verschiedene Anwendungsgebiete. Da diese Werkstoffe sehr schwer verformbar sind, kommt nur eine Formgebung durch Gießen in Frage. Insbesondere beim Gießen aber werfen titanhaltige Metalle weitere Probleme auf, auf die nachfolgend noch näher eingegangen werden wird.

Einige Beispiele für den Einsatz titanhaltiger Werkstoffe werden wie folgt angegeben:
Ventile für Verbrennungskraftmaschinen
Turbinenräder und Turbinenschaufeln
Verdichterräder
Biomedizinische Prothesen (Implantate)
Kompressorgehäuse im Flugzeugbau.

Insbesondere im Motor-Rennsport haben sich sowohl Einlaß- als auch Auslaßventile aus bestimmten Titanlegierungen hervorragend bewährt, so daß an einen Masseneinsatz für Verbrennungskraftmaschinen aller Art gedacht wird.

In dem Aufsatz von Schädlich-Stubenrauch u.a. "Numerical simulation of the alpha case as a quality criterion for the investment casting of small, thin-walled titanium parts", veröffentlicht auf der Sixth World Conference on Titanium", Frankreich 1988, Seiten 649 bis 654, sind die Probleme beschrieben, die dann entstehen, wenn Titanlegierungen in Formen aus Oxidwerkstoffen vergossen werden. Dabei bilden sich nicht nur auf der Oberfläche des Gußteils Titanoxide, sondern bis zu 10 Gewichtsprozent Sauerstoff gehen auch an den Korngrenzen in Lösung, so daß sich die Notwendigkeit ergibt, die Oberflächen der Gußteile nachträglich zu bearbeiten, was auf chemische oder spanabhebende Weise geschehen kann. Notwendigerweise wächst die Dicke der sauerstoffhaltigen Oberflächenschicht mit zunehmender Abkühldauer, so daß die Verwendung von Formen aus oxidischen Werkstoffen auf dünnwandige Werkstücke begrenzt ist. Außerdem wird angegeben, daß es zweckmäßig ist, die fertig bearbeiteten Werkstücke nachträglich einem isostatischen Heißpreßverfahren (HIP-Verfahren) auszusetzen. Dadurch verteuern sich die Kosten der Bauteile ganz erheblich. Der Aufsatz untersucht diese Zusammenhänge anhand von keilförmigen Gußteilen.

Durch den Aufsatz von Tsutomu Oka u.a. "Manufacturing of automotive engine valves by plasma package melting of titanium scraps", veröffentlicht auf der Sixth World Conference on Titanium, Frankreich, 1988, Seiten 621 bis 626, ist es bekannt, Ventile für Verbrennungsmotoren aus Titanlegierungen herzustellen. Für die Herstellung von Einlaßventilen, die bei relativ niedrigen Betriebstemperaturen bis zu etwa 450 °C arbeiten, wird die Legierung Ti 6 Al 4 V empfohlen. Für die Auslaßventile, deren Betriebstemperaturen bis über 700 °C hinausgeht, wird die Legierung Ti 6 Al 2 Sn 4 Zr 2 Mo 0,1 Si empfohlen, wobei darauf hingewiesen wird, daß es schwierig ist, Teile mit einem Durchmesser unterhalb 10 mm herzustellen, weil dieser Werkstoff schwierig zu bearbeiten ist. Es wird daher weiterhin empfohlen, für die Auslaßventile die Ventilteller aus der zuletzt genannten Titanlegierung herzustellen und mit Ventilschäften zu vereinigen, die aus Ti 6 Al 4 V bestehen. Auch diese Veröffentlichung zeigt, welche Umwege eingeschlagen werden müssen, um bei der Verarbeitung den Stoffeigenschaften bestimmter Titanlegierungen entgegenzukommen.

Durch den Aufsatz von Zwicker u.a. "Evaluation of centrifugally cast TiAl5Fe2.5 alloy for implant material" ist es bekannt, Hüftgelenk-Prothesen bzw. -Implantate aus der im Titel angegebenen Titanlegierung durch ein Schleudergußverfahren in einer Kupferkokille herzustellen. Einerseits wird angegeben, daß durch die hohe Abschreckgeschwindigkeit durch das Kupfer eine vorteilhafte feine Kornstruktur erreicht wird, andererseits wird jedoch direkt darauf hingewiesen, daß die hohe Abkühlgeschwindigkeit zur Ausbildung von Poren durch Gaseinschlüsse führt sowie zur Ausbildung von Schrumpf-Hohlräumen, die zu einer Kerbwirkung führen. Es wird daher empfohlen, die Poren und Lunker durch ein HIP-Verfahren zu beseitigen, wobei jedoch ausdrücklich darauf hingewiesen wird, daß es selbst mit einem Druck von 1.000 bar nur möglich ist kleine Poren und Lunker zu schließen, nicht aber Poren an der Werkstückoberfläche, die durch den Druck geöffnet werden und die Kerbwirkung noch verstärken. Um diese Defekte zu vermeiden, wird angegeben, die Oberflächenunregelmäßigkeiten durch Schweißen zu schließen, womit allerdings wieder der Nachteil einer groben Kornstruktur erkauft wird. Als Parameter für das HIP-Verfahren wird eine Einwirkungsdauer eines Drucks von 1.000 bar über 3 Stunden bei 950 °C angegeben. Die Veröffentlichung enthält den weiteren Hinweis, daß die Kupfer-Kokille im Verhältnis zum Werkstück-Gewicht ein relativ sehr hohes Gewicht aufweisen muß, um eine Reaktion zwischen der flüssigen Titanlegierung und der Kupferoberfläche zu vermeiden. Dieser Hinweis läßt nur den Schluß zu, daß die Kupferkokille in kaltem Zustand eingesetzt werden muß, daß also jegliche Vorwärmung der Kupfer-Kokille zu unterbleiben hat, womit wiederum eine unerwünscht hohe Abschreckgeschwindigkeit verbunden ist.

Aus dem nachgewiesenen Stande der Technik läßt sich herleiten, daß an die Auswahl des Formwerkstoffs, d.h. des Werkstoffs für die Gießform, extrem hohe Anforderungen zu stellen sind, und daß ferner Verarbeitungsrichtlinien in engen Grenzen einzuhalten sind, soll es nicht zu einer Schädigung des Werkstücks oder Kokille bzw. Gießform kommen. Es stehen sich also gewissermaßen die Eigenschaften der Schmelze und die der Gießform diametral entgegen, wobei zu beachten ist, daß die meisten Titanlegierungen bei Temperaturen vergossen werden müssen, die deutlich oberhalb von 1.500 °C liegen, während Kupfer einen Schmelzpunkt von 1.084 °C hat und der eutektische Punkt der Legierung Kupfer/Titan bei 865 °C liegt.

Die EP-0 443 544 B1 befaßt sich mit dem Problem, die Formgenauigkeit bzw. Formtreue von Schleudergußkokillen aus Kupfer und die Entformbarkeit der Werkstücke aus Titan-Legierungen dadurch zu verbessern, daß man dem Kupfer als Legierungselemente Zirkonium, Chrom, Beryllium, Kobalt und Silber zusetzt, wobei die Summe aller Legierungselemente jedoch nicht über 3 Gewichtsprozent hinausgeht. Ein Vergleichsbeispiel, bei dem das Kupfer mit 18 Gewichtsprozent Nickel legiert wurde, hat nicht zum Erfolg geführt. Die betreffende Druckschrift befaßt sich zwar mit der elektrischen Leitfähigkeit des Werkstoffs, nicht aber mit dessen thermischer Leitfähigkeit, so daß die Probleme der hohen Abschreckgeschwindigkeit, der Lunker- und der Porenbildung nicht behandelt werden. Andererseits geht aber auch diese Literaturstelle auf die Nachteile keramischer bzw. oxidischer Formwerkstoffe ein.

Durch die Aufsätze von:
1. Krone, "Herstellung und Eigenschaften von Fein- und Kompaktgußteilen aus Titanwerkstoffen" veröffentlicht in "GIESSEREI 65" (28. September 1978) Nr. 20, Seiten 540 bis 549, und
2. Krone u.a., "Titanium Castings: Manufacture and Properties", veröffentlicht in "AFS International Cast Metals Journal" (März 1977 2., Nr. 1, Seiten 37 bis 40,
   ist es bekannt, Feingußformen mit einer "metallischen Frontschicht" herzustellen, die aber sogenannte "verlorene Formen" nach dem Wachs-Ausschmelzverfahren für einmalige Verwendung sind und hinter der metallischen Frontschicht nichtmetallische, vor allem oxidische, Werkstoffe besitzen. Auch die metallische Frontschicht wird aus einer Paste (slurry) aus Metallpulvern, darunter Wolfram-, Tantal-, Niob und/oder Molybdänpulver, Inhibitorbildnern und flüssigen metallorganischen Verbindungen hergestellt und gebrannt, d.h., sie enthält merkliche Anteile an nichtmetallischen Beimengungen. Die kompletten Formen sollen durch Wasserstrahlen, Schleuderradputzen, Sandstrahlen etc. von den Gußteilen entfernt werden, die danach noch durch Salzbäder und manuelles Putzen gereinigt werden müssen. Dies funktioniert nur deshalb, weil auch die Frontschicht durch die nichtmetallische Beimengungen keinen festen Zusammenhalt besitzt.

Ein entscheidender Unterschied liegt aber darin, daß die bekannte Frontschicht nicht die Aufnahme von Sauerstoff in die Gußteile verhindern kann, weil einerseits die Frontschicht selbst Sauerstoffverbindungen enthält, für Sauerstoff aus der nachfolgenden Keramikmasse durchlässig ist und insbesondere beim Abguß eine sehr hohe Temperatur annimmt, die die Wanderung von Sauerstoff begünstigt.

Der Erfindung liegt daher die Aufgabe zugrunde, ein Verfahren und eine Gießform der eingangs beschriebenen Gattung anzugeben, durch das bzw. die Gußteile aus Titan oder Titanlegierungen erhalten werden, die eine glatte Oberfläche ohne Sauerstoffaufnahme aufweisen, und die frei von Lunkern und anderen Hohlräumen sind, so daß aufwendige Nachbearbeitungen, die zu einer Beseitigung der bekannten Fehler führen, zumindest weitgehend entfallen können.

Die Lösung der gestellten Aufgabe erfolgt daher bei dem eingangs beschriebenen Verfahren erfindungsgemäß durch die Merkmale im kennzeichen des Patentanspruchs 1

Durch die Verwendung einer solchen Gießform ist jegliche Reaktion des Formwerkstoffs mit dem Gußwerkstoff ausgeschlossen, und es unterbleibt ein Anschmelzen der Oberfläche des Formhohlraums unter dem Einfluß der Schmelzentemperatur selbst dann, wenn die Gießform vor dem Abguß bereits eine Temperatur aufweist, die deutlich oberhalb 800 °C liegt.

Weiterhin wird durch die Verwendung von Metallen aus der Gruppe Tantal, Niob, Zirkonium und/oder deren Legierungen eine wesentlich geringere Abschreckgeschwindigkeit erzielt, weil diese Werkstoffe eine deutlich geringere Wärmeleitfähigkeit aufweisen. So beträgt beispielsweise die Wärmeleitfähigkeit von Tantal und von Niob 14 % bzw. 13 % der Wärmeleitfähigkeit von Kupfer, und die Wärmeleitfähigkeit von Zirkonium beträgt gar nur 6 % derjenigen von Kupfer. Auch die spezifische Wärmekapazität der angegebenen Formwerkstoffe ist deutlich geringer als diejenige von Kupfer: So beträgt beispielsweise die spezifische Wärmekapazität von Tantal 36 % des vergleichbaren Wertes von Kupfer, und die spezifischen Wärmekapazität der Werkstoffe Niob und Zirkonium betragen 70 % bzw. 72 % des vergleichbaren Wertes von Kupfer. Diese Eigenschaften führen, insbesondere in ihrer Verknüpfung, zu einer deutlich verzögerten Abkühlung der Gußteile, so daß die gefürchtete Ausbildung einer harten Schale mit im Innern befindlichen Lunkern und Poren unterbleibt. Die Schmelze hat hinreichend Zeit, um während der Schrumpfung des Gußteils beim Abkühlen und Erstarren zu folgen.

Durch die erfindungsgemäße Werkstoffauswahl werden Nachbearbeitungen der Werkstückoberfläche zumindest weitgehend vermieden, sei es durch Entfernen der Randschicht, sei es durch örtliche Schweißarbeiten, und auch eine Nachverdichtung der Werkstücke durch das sogenannte HIP-Verfahren ist überflüssig geworden. Es ist bei Ventilen für Verbrennungsmotoren auch nicht mehr erforderliche, Ventilteller und Schaft aus unterschiedlichen Werkstoffen zusammenzusetzen, nur weil die Verarbeitung dieser Werkstoffe schwierig ist.

Es ist dabei nicht erforderlich, daß die Gießform insgesamt aus den ausgewählten Werkstoffen besteht, sondern es ist im Grenzfall auch nur notwendig, die mit der Schmelze in Berührung kommende Oberfläche aus den genannten Metallen oder ihren Legierungen auszubilden, wobei im Grenzfall bereits eine Schichtdicke von 2 mm ausreichend ist. Eine solche Oberfläche wird nachfolgend als "Schale" bezeichnet.

Besonders vorteilhaft ist hierbei die Verwendung einer Tantalbasislegierung.

Das erfindungsgemäße Verfahren ermöglicht insbesondere die Herstellung einwandfreier Gußteile im Schleudergußverfahren.

Die Abschreckgeschwindigkeit wird auch dadurch verringert, daß die Gießform vor dem Abguß vorgewärmt wird, wobei die Vorwärmtemperatur unterhalb der Liquidustemperatur der zu vergießenden Schmelze eingestellt wird. Besonders vorteilhaft ist es hierbei, wenn die Vorwärmtemperatur zwischen 800 °C und der Solidustemperatur der zu vergießenden Schmelze eingestellt wird.

Im Hinblick auf eine fehlerfreie Ausbildung der Gußteile ist es im Zuge einer weiteren Ausgestaltung der Erfindung besonders vorteilhaft, wenn der Abguß der Schmelze in die Gießform in einer geschlossenen Formkammer bei einem Druck von weniger als 100 mbar, vorzugsweise von weniger als 10 mbar durch geführt wird.

Andererseits kann zur Erhöhung der Abkühlgeschwindigkeit der Gießform nach dem Abguß bzw. nach dem Erstarren der Gußteile mit besonderem Vorteil ein Inertgas, vorzugsweise mindestens ein Edelgas aus der Gruppe Argon und Helium, in die Formkammer eingeleitet werden, um die Zyklusdauer zu verringern.

Die Abkühlgeschwindigkeit ist dabei - unter Aufrechterhaltung der Rotationsbewegung der Gießform - um so größer, je höher der Druck des Inertgases in der Formkammer ist, wobei das Inertgas unter einem Druck zwischen 100 mbar und Atmosphärendruck gesetzt werden kann. Besonders vorteilhaft ist es hierbei, wenn das Inertgas in der Formkammer unter einen überatmosphärischen Druck gesetzt wird.

Die Erfindung betrifft auch eine Gießform nach dem Oberbegriff des Patentanspruchs 8. Zur Lösung der gleichen Aufgabe ist diese Gießform erfindungsgemäß gekennzeichnet durch die Merkmale im kennzeichen des Patentanspruchs 8.

Besonders vorteilhaft ist es hierbei, wenn der Formwerkstoff oder die Schale mindestens 50 Gewichtsprozent Tantal enthält. Dem Formwerkstoff können weitere vorteilhafte Eigenschaften dadurch verliehen werden, daß man zu dem Tantal noch weitere Metalle aus der Gruppe Titan, Hafnium, Wolfram und/oder Vanadium hinzulegiert. Bei Verwendung eines Formwerkstoffes mit mindestens 50 Gewichtsprozent Tantal ist es besonders vorteilhaft, wenn der Formwerkstoff noch mindestens eines der Metalle Titan, Zirkonium und Wolfram enthält, wobei die Summe der Anteile dieser Metalle jedoch 30 Gewichtsprozent nicht überschreiten sollte. Analoge Überlegungen gelten natürlich auch für die Zusammensetzung der "Schale".

Die Gießformen müssen nämlich nicht aus einem homogenen Formwerkstoff aus den erfindungsgemäßen Metallen oder Legierungen bestehen, sondern es können in einem Grundkörper auch den Formhohlraum begrenzende Schalen bzw. rein metallische Überzüge aus den besagten Metallen angeordnet sein, während der Grundkörper selbst aus anderen Stoffen besteht.

Es ist besonders vorteilhaft, wenn bei einem Überzug des Formhohlraums der Gießform durch Schalen aus mindestens einem von nichtmetallischen Beimengungen freien Metall aus der Gruppe Tantal, Niob, Zirkonium und/oder deren Legierungen die Dicke der Schalen mindestens 2 mm beträgt und der Grundkörper der Gießform entweder:
a) aus mindestens einem der Metalle Eisen, Nickel und/oder deren Legierungen, vorzugsweise aus Eisenbasislegierungen, Nickelbasislegierungen, austenitischen hitzebeständigen Stählen oder
b) aus Titan, Titanlegierungen, Titanaluminid oder
c) aus mindestens einem sauerstoffreien nichtmetallischen Werkstoff wie Graphit und Siliziumnitrid besteht.

Grundkörper aus den Stoffen gemäß b) und c) sind wegen ihrer geringeren Masse besonders gut für Schleudergießform geeignet.

Weitere vorteilhafte Ausgestaltungen des Erfindungsgegenstandes ergeben sich aus den übrigen Unteransprüchen.

Drei Ausführungsbeispiele des Erfindungsgegenstandes werden nachfolgend anhand der Figuren 1 bis 7 näher erläutert.

Es zeigen:
- Figur 1: einen teilweisen Vertikalschnitt durch eine Vorrichtung mit einem stationären Kaltwandtiegel mit Bodenauslaß und einer Gießform, die als Schleudergießform mit einer Vielzahl von Formhohlräumen ausgebildet ist,
- Figur 2: einen Axialschnitt durch die Gießform nach Figur 1,
- Figur 3: einen Radialschnitt durch die Gießform nach Figur 2,
- Figur 4: einen Axialschnitt durch ein Ventil eines Verbrennungsmotors, hergestellt in einer herkömmlichen Kupfer-Kokille,
- Figur 5: einen Axialschnitt analog Figur 4 durch ein Ventil eines Verbrennungsmotors, hergestellt in einer erfindungsgemäßen Gießform,
- Figur 6: eine Variante der Vorrichtung nach Figur 1 mit einem Kipptiegel unter Darstellung weiterer Einzelheiten und
- Figur 7: einen vergrößerten Axialschnitt durch eine Gießform, bei der der Grundwerkstoff der Gießform aus einem anderen Material beseht.

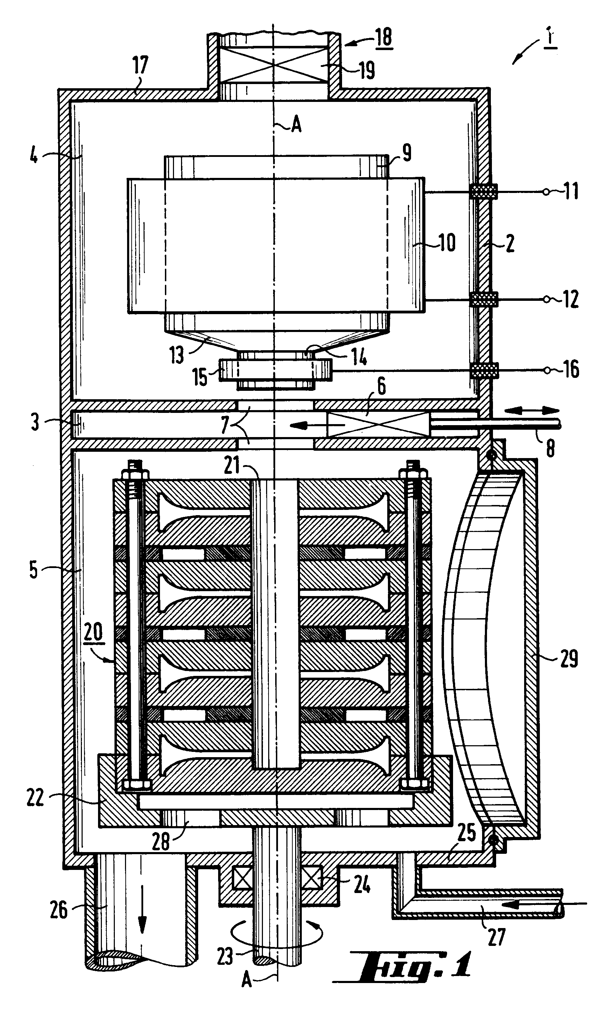

Figur 1 zeigt eine Gießvorrichtung 1 mit einem druckfesten und gasdichten Gehäuse 2, dessen Innenraum durch ein Schiebergehäuse 3 in eine Schmelzkammer 4 und eine Formkammer 5 unterteilt ist. Im Schiebergehäuse 3 befindet sich ein Schieber 6, durch den zwei fluchtende Gießöffnungen 7 mittels einer Antriebsstange 8 verschließbar sind.

Im Innern der Schmelzkammer 4 befindet sich ein Kaltwandtiegel 9, dessen Inhalt, der Gußwerkstoff, durch eine induktive Heizeinrichtung 10 aufgeschmolzen werden kann. Für die Versorgung mit Schmelzenergie dienen zwei Stromanschlüssse 11 und 12. Der Kaltwandtiegel 9 besitzt in seinem Boden 13 einen Bodenauslaß 14, der durch eine Verschlußeinrichtung 15, die als Magnetspule ausgebildet sein kann, freigebbar und wieder verschließbar ist. Der Strom für die Verschlußeinrichtung 15 wird über einen Anschluß 16 zugeführt. In die Decke 17 der Schmelzkammer 4 mündet eine Chargiereinrichtung 18, von der nur das untere Chargierventil 19 angedeutet ist. Bau- und Betriebsweise eines solchen Kaltwandtiegels sind bekannt und werden daher nicht näher beschrieben. Es sei nur so viel gesagt, daß sich in einem Kaltwandtiegel ein sogenannter "Skull" ausbildet, der eine Reaktion der Schmelze mit dem Tiegelmaterial verhindert. Die Anschlüsse für einen Kühlmittelkreislauf sind der Einfachheit halber fortgelassen.

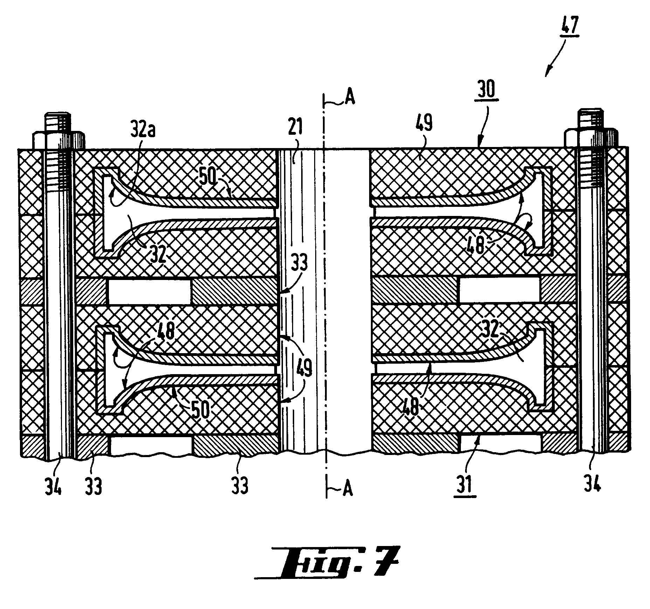

Es versteht sich, daß der beschriebene, induktiv beheizte Kaltwandtiegel auch durch einen solchen ersetzt werden kann, der mittels eines Lichtbogens, einer Elektronenstrahl- oder Plasmakanone beheizt werden kann. Auch ist es nicht erforderlich, die Schmelze durch einen Bodenauslaß abzugießen; vielmehr ist es möglich, am oberen Tiegelrand einen Überlauf, eine sogenannte Gießlippe, vorzusehen. In einem solchen Fall wird der Kaltwandtiegel zweckmäßigerweise in einem sogenannten Kippstuhl aufgehängt und über eine Drehachse entleert, die sich in der Nähe des Überlaufs befindet. Eine solche Vorrichtung zeigt Figur 6.

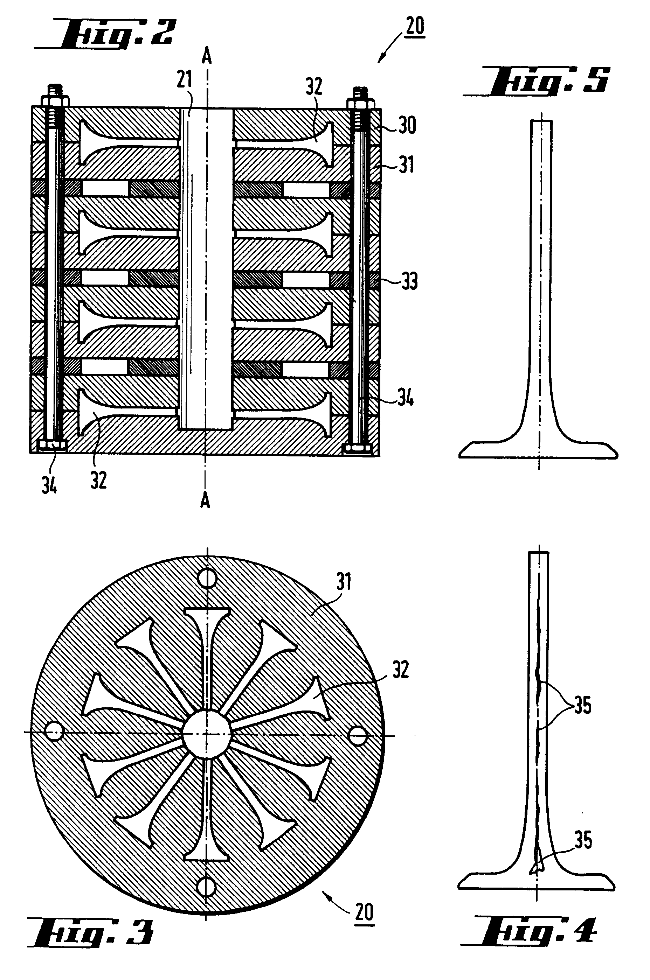

In der Formkammer 5 befindet sich eine Gießform 20, deren Einzelheiten anhand der Figuren 2 und 3 nachstehend noch näher erläutert werden. Es sei hier nur so viel ausgeführt, daß die Gießform 20 einen Eingußkanal 21 besitzt, der konzentrisch zu einer senkrechten Achse A-A ausgerichtet ist, die mit der Rotationsachse der Gießform 20 und der Tiegelachse übereinstimmt. Die Gießform 20 ist konzentrisch in einem Schleuderteller 22 gehalten, der durch eine Schleuderwelle 23 angetrieben werden kann, die mittels einer gasdichten Drehdurchführung 24 durch einen Boden 25 der Formkammer 5 hindurchgeführt ist. Die Formkammer 5 ist an eine Saugleitung 26 angeschlossen, die zu einem Vakuum-Pumpsatz führt, der aus mindestens einer Vakuumpumpe, vorzugsweise aber aus einer Reihenschaltung von Vakuumpumpen besteht, die für unterschiedliche Druckbereiche ausgelegt sind. In die Formkammer 5 mündet weiterhin eine Gasleitung 27, durch die Inertgas zu Kühlzwecken der Gießform 20 eingeführt werden kann. Öffnungen 28 im Schleuderteller 22 erleichtern den Austausch der Kühlgase auch auf der Unterseite der Gießform 20. Die Formkammer 5 ist weiterhin mit einer Tür 29 versehen, die zum Einsetzen und zum Herausnehmen der Gießform 20 dient. Es sei darauf verwiesen, daß Figur 1 nur eine sehr schematische Darstellung der gesamten Gießvorrichtung zeigt.

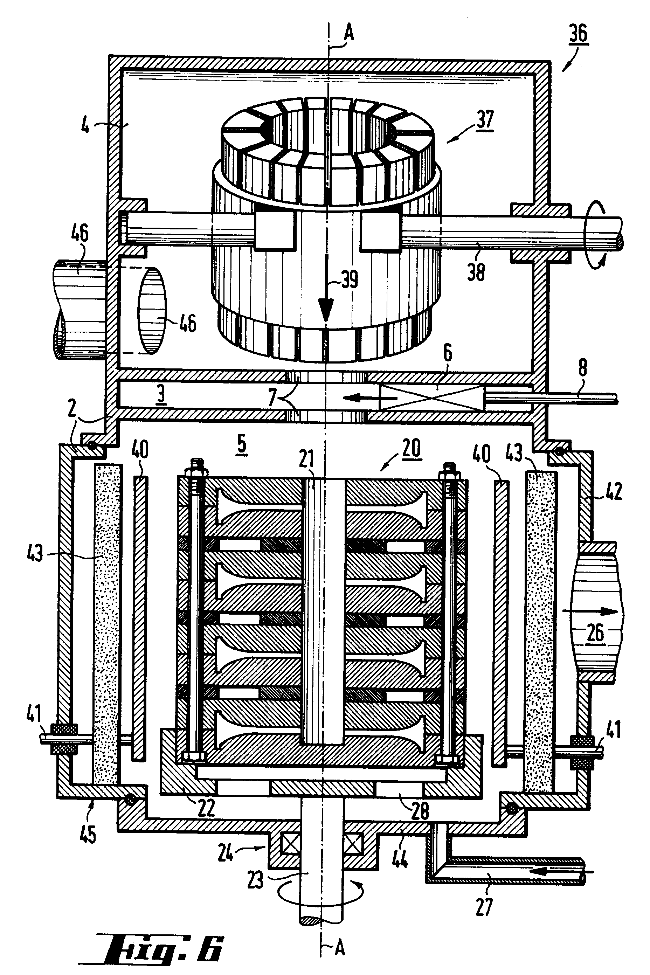

Die Gießform 20 nach den Figuren 2 und 3 besteht aus einem Stapel paarweise und spiegelbildlich zueinander angeordneter Scheiben 30 und 31, die Formhohlräume 32 zwischen sich einschließen, die im vorliegenden Fall dem in Figur 5 gezeigten Ventil eines Verbrennungsmotors entsprechen. Die Scheiben 30 und 31 sind zur Rotationsachse A-A koaxial ausgebildet, und sämtliche Formhohlräume 32 sind an den gemeinsamen, gleichfalls in der Rotationsachse A-A liegenden Eingußkanal 21 angeschlossen.

Jeweils ein Scheibenpaar 30/31 ist durch Distanzhalter 33 von dem benachbarten Scheibenpaar getrennt, wobei der Distanzhalter 33 auch den Eingußkanal 21 auf dem Umfang abdichtet. Dadurch stehen nur die inneren Enden der Formhohlräume 32 mit dem Eingußkanal 21 in Verbindung. An dieser Stelle liegt das Ende der Ventilschäfte, die nach Beendigung von Gieß-und Abkühlphase von dem Material abgetrennt werden müssen, das sich in dem Eingußkanal 21 befindet. Die stapelförmige Anordnung von Scheiben 30 und 31 sowie der Distanzhalter 33 wird durch vier äquidistant auf den Umfang verteilte Zuganker 34 zusammengehalten.

Mit einer Gießform 20 nach den Figuren 2 und 3 können gleichzeitig 40 Ventile gemäß Figur 5 hergestellt werden.

Durch die Einwirkung des Kühlgases bei fortgesetzter Rotationsbewegung der Gießform 20 kann auch eine gerichtete Erstarrung der Gußteile durchgeführt werden, und zwar ausgehend vom Außenumfang der Gießform, da an dieser Stelle die Einwirkung des Kühlgases am intensivsten ist.

Mit einer Schleudergießform 20 nach den Figuren 2 und 3 wurden beispielsweise Auslaßventile für Verbrennungsmotoren nach Figur 5 hergestellt. Der Formwerkstoff bestand aus einer Legierung aus 90 Gewichtsprozent Tantal und 10 Gewichtsprozent Wolfram. Der in Figur 5 gezeigte Axialschnitt durch das Ventil läßt keinerlei Lunker, Hohlstellen oder andere Porositäten erkennen; auch die Oberfläche war von einwandfreier Beschaffenheit.

Beim Ersatz der Scheiben 30 und 31 aus der angegebenen Tantal-Legierung durch Scheiben aus Kupfer oder einer Kupferlegierung mit einem hohen Kupferanteil ließen sich aus der gleichen Titanlegierung nur Ventile gemäß Figur 4 erzeugen, die in der Schliffebene entlang nahezu der gesamten Längsachse Lunker und Hohlräume 35 aufwiesen.

### Beispiel 1:

In einer Vorrichtung nach Figur 1 wurde im Kaltwandtiegel 9 die Legierung 50 Ti 46 Al 2 Cr 2 Nb bei einem Druck von 10⁻¹ mbar in der Schmelzkammer 4 erschmolzen und nach dem Aufschmelzen für die Dauer von 10 Minuten homogenisiert. Im Anschluß daran wurde die Schmelze bei einer Schmelzentemperatur von 1.540 °C bei einem Druck von 10⁻¹ mbar in die Gießform 20 in der Formkammer 5 abgegossen. Die Gießform 20 war zuvor mittels nicht dargestellter Heizeinrichtungen auf eine Temperatur von 1.400 °C aufgeheizt worden. Während des Gießens rotierte die Gießform mit 1.000 Umdrehungen pro Minute. Nach Beendigung des Abgusses wurde der Schieber 6 geschlossen. Ca. 20 Sekunden nach Beendigung des Abgusses wurde in die Formkammer 5 über die Gasleitung 27 Argon eingelassen, bis ein Druck von 1000 mbar erreicht wurde. Die Rotationsbewegung der Gießform 20 wurde bis zur völligen Erstarrung der Gußteile fortgesetzt. Nach etwa 60 Minuten waren die Gußteile vollständig erstarrt, und die Gießform wurde der Formkammer 5 entnommen. Die axialen Schliffbilder der einzelnen Ventile entsprachen denjenigen nach Figur 5. Das Schliffbild zeigte keine sichtbaren Poren oder Lunker, so daß eine Nachverdichtung, beispielsweise durch ein HIP-Verfahren, nicht erforderlich war.

### Beispiel 2:

Der Versuch nach Beispiel 1 wurde wiederholt, jedoch mit den Unterschieden, daß an die Stelle der Gießform 20 aus Scheiben aus einer Tantal-Wolfram-Legierung Scheiben aus Reinkupfer traten, die infolge der Eigenschaften dieses Werkstoffes nicht ausreichend vorgeheizt werden konnten. Bei Beginn des Abgusses hatte die Kokille mithin Raumtemperatur. Nach dem Entnehmen der einzelnen Ventile aus der Kupferkokille zeigten die Schliffbilder sämtlich das Aussehen nach Figur 4, d.h. entlang der Ventilachse befanden sich typische Lunker und andere poröse Stellen, so daß derartige Ventile entweder verworfen oder durch ein HIP-Verfahren nachverdichtet werden mußten.

Figur 6 zeigt eine Gießvorrichtung 36, die eine Variante der Vorrichtung nach Figur 1 ist. Gleiche Teile oder Teile mit gleicher Funktion sind mit gleichen Bezugsziffern versehen: In der Schmelzkammer 4 befindet sich ein induktiv beheizbarer Kaltwandtiegel 37, der als Kipptiegel ausgebildet ist und durch eine Kippwelle 38 in Kippstellung gebracht werden kann, in der die Schmelze über den Rand in Richtung des Pfeils 39 durch die Gießöffnungen 7 in die Gießform 20 abgegossen werden kann. Die angetriebene Kippwelle dient gleichzeitig zur Zuführung von Kühlwasser und Schmelzstrom, jedoch sind die entsprechenden Leitungen nicht dargestellt.

Die Gießform 20 ist von einem ortsfesten Heizzylinder 40 umgeben, dessen Stromzuführungen 41 durch die Wand 42 der Formkammer 5 hindurchgeführt sind und der von einer zylindrisch ausgebildeten Wärmedämmung 43 umgeben ist. In diesem Fall besitzt die Formkammer 5 einen Boden 44, der zusammen mit dem Schleuderteller 22 und dessen Antrieb abgesenkt werden kann, was naturgemäß nach dem Fluten und ausreichender Abkühlung geschieht. Dadurch gelangt die Oberseite der Gießform 20 in eine Stellung unterhalb der Unterkante 45 der Formkammer 5, so daß die Gießform 20 vom Schleuderteller 22 abgehoben werden kann. Um die Schmelzkammer 4 ständig unter Vakuum zu halten, ist diese über eine weitere Saugleitung 46 mit dem Vakuumpumpsatz verbunden.

Figur 7 zeigt den oberen Teil einer wiederverwendbaren Gießform 47 analog der Gießform 20 nach Figur 2, jedoch mit dem Unterschied, daß die Formhohlräume 32 in den Scheiben 30 und 31 von Schalen 48 (einfach schraffiert) umgeben sind, die aus einem von nichtmetallischen Beimengungen freien Metall aus der Gruppe Tantal, Niob, Zirkonium und/oder deren Legierungen bestehen. Dies besagt, daß die Schalen 48 - jeweils für sich - dicht, kompakt, homogen und fest zusammenhängend sind, also z.B. nicht durch Wasserstrahlen, Bürsten oder dergleichen entfernt werden können.

Die Ausdrücke "Schale" oder "Überzug" besagen, daß deren Metall entweder fest mit einem Grundkörper 49 (kreuzschraffiert) zusammenhängen kann, der den Scheiben ihre Grundfestigkeit verleiht, oder auswechselbar in den Grundkörper 49 eingesetzt sein kann. Auswechselbare Schalen 48 können beispielhaft entweder durch Gießen, mechanische Bearbeitung oder Explosionsverformung hergestellt werden, fest mit dem Grundkörper 49 verbundenen Schalen 48 beispielhaft durch Flammspritzen, galvanische Prozesse, Aufsintern oder dergleichen.

Dieser Grundkörper 49 kann aus mindestens einem der Metalle Kupfer, Eisen, Nickel, Titan, oder deren Legierungen, sowie aus Titanaluminid bestehen, vorzugsweise aus Eisenbasislegierungen, Nickelbasislegierungen, austenitischen hitzebeständigen Stählen, oder dem besagten Titan, Titanlegierungen oder Titanaluminid. Der Grundkörper 49 kann aber auch aus mindestens einem nichtmetallischen sauerstoffreien Werkstoff wie beispielsweise aus Graphit und Siliziumnitrid bestehen.

Für Schleuder-Gießformen sind insbesondere Titan, Titanlegierungen, Titanaluminide, Graphit und/oder Siliziumnitrid sehr gut als Werkstoffe für den Grundkörper 49 geeignet, weil die Masse des Grundkörpers 49 überwiegt und dadurch die rotierenden Massen gering gehalten werden können. Außerdem haben diese Werkstoffe, insbesondere im Verbund mit den Schalen 48 eine hohe Festigkeit und Hitzebeständigkeit.

Der Grundkörper 49 ist für die Aufnahme oder Einbettung der Schalen 48 mit Ausnehmungen 50 versehen. Die mit der Schmelze in Berührung kommende Oberfläche der Schalen bzw. des Formhohlraums 32 ist mit 32a bezeichnet.

Metallische Grundkörper 49 können beispielhaft durch Gießen und/oder mechanische Bearbeitung oder Verformung hergestellt werden, nichtmetallische Grundkörper 39 beispielhaft durch Pressen in Formen und nachfolgendes Sintern und gegebenenfalls nachfolgende mechanische Bearbeitung.

## Patentansprüche

1. Verfahren zum Herstellen von Gußteilen aus einer Schmelze eines reaktiven Metalls aus der Gruppe Titan, Titanlegierungen und Titanbasislegierungen in einer nicht-reaktiven Atmosphäre und in einer wiederverwendbaren Gießform (20, 47), die als Schleudergießform ausgebildet ist und eine Rotationsachse (A-A) und einen zentralen Eingußkanal (21) aufweist, der mit einer Vielzahl von Formhohlräumen (32) in Verbindung steht, die von der Rotationsachse (A-A) weggerichtet sind, **dadurch gekennzeichnet,** daß die Abschreckgeschwindigkeit der Schmelze dadurch begrenzt wird, daß
a) eine Gießform (20, 47) verwendet wird, die mindestens an ihrer mit der Schmelze in Berührung kommenden Oberfläche (32a) aus mindestens einem von nichtmetallischen Beimengungen freien Metall aus der Gruppe Tantal, Niob, Zirkonium und/oder deren Legierungen besteht, und daß
b) die Gießform (20, 47) vor dem Abguß vorgewärmt wird.

2. Verfahren nach Anspruch 1, **dadurch gekennzeichnet,** daß eine Gießform (20, 47) verwendet wird, die mindestens an ihrer mit der Schmelze in Berührung kommenden Oberfläche aus einer Tantalbasislegerung besteht.

3. Verfahren nach Anspruch 1, **dadurch gekennzeichnet,** daß die Vorwärmtemperatur unterhalb der Liquidustemperatur der zu vergießenden Schmelze eingestellt wird.

4. Verfahren nach Anspruch 3, **dadurch gekennzeichnet,** daß die Vorwärmtemperatur zwischen 800°C und der Solidustemperatur der zu vergießenden Schmelze eingestellt wird.

5. Verfahren nach Anspruch 1, **dadurch gekennzeichnet,** daß der Abguß der Schmelze in die Gießform (20, 47) in einer geschlossenen Formkammer (5) bei einem Druck von weniger als 100 mbar, vorzugsweise von weniger als 10 mbar, durchgeführt wird.

6. Verfahren nach Anspruch 1, **dadurch gekennzeichnet,** daß zur Erhöhung der Abkühlgeschwindigkeit der Gießform (20, 47) nach dem Abguß ein Inertgas, vorzugsweise mindestens ein Edelgas aus der Gruppe Argon und Helium, in die Formkammer (5) eingeleitet wird.

7. Verfahren nach Anspruch 6, **dadurch gekennzeichnet,** daß das Inertgas in der Formkammer (5) unter einen Druck zwischen 100 mbar und Atmosphärendruck gesetzt wird.

8. Gießform zum Herstellen von Gußteilen aus einer Schmelze eines reaktiven Metalls aus der Gruppe Titan, Titanlegierungen und Titanbasislegierungen, die als wiederverwendbare Schleudergießform ausgebildet ist und eine Rotationsachse (A-A) und einen zentralen Eingußkanal (21) aufweist, der mit einer Vielzahl von Formhohlräumen (32) in Verbindung steht, die von der Rotationsachse (A-A) weggerichtet sind,
**dadurch gekennzeichnet,** daß der Formwerkstoff der Gießform (20, 47) mindestens an der mit der Schmelze in Berührung kommenden Oberfläche (32a) aus mindestens einem von nichtmetallischen Beimengungen freien Metall aus der Gruppe Tantal, Niob, Zirkonium und/oder deren Legierungen besteht.

9. Gießform nach Anspruch 8, **dadurch gekennzeichnet,** daß der Formwerkstoff mindestens an der mit der Schmelze in Berührung kommenden Oberfläche (32a) aus einer Tantalbasislegierung besteht.

10. Gießform nach Anspruch 9, **dadurch gekennzeichnet,** daß der Formwerkstoff mindestens 50 Gewichts-% Tantal enthält.

11. Gießform nach Anspruch 8, **dadurch gekennzeichnet,** daß der Formwerkstoff noch mindestens ein weiteres Metall aus der Gruppe Titan, Hafnium, Wolfram, Vanadium enthält.

12. Gießform nach Anspruch 8, **dadurch gekennzeichnet,** daß der Formwerkstoff außer mindestens 50 Gewichts-% Tantal mindestens eines der Metalle Titan, Zirkonium und Wolfram enthält.

13. Gießform nach Anspruch 12, **dadurch gekennzeichnet,** daß der Anteil der Metalle Titan, Zirkonium und Wolfram in der Summe maximal 30 Gewichts-% beträgt.

14. Gießform nach Anspruch 11, **dadurch gekennzeichnet,** daß die Formhohlräume (32) der Gießform (47) von Schalen (48) umgeben sind, die aus mindestens einem von nichtmetallischen Beimengungen freien Metall aus der Gruppe Tantal, Niob, Zirkonium und/oder deren Legierungen bestehen und die in einem Grundkörper (49) der Gießform (47) angeordnet sind, der aus mindestens einem der Metalle, Eisen, Nickel Titan, Titanaluminid und/oder deren Legierungen, vorzugsweise aus Eisenbasislegierungen, Nickelbasislegierungen oder austenitischen hitzebeständigen Stählen, Titan, Titanlegierungen oder Titanaluminid besteht.

15. Gießform nach Anspruch 11, **dadurch gekennzeichnet,** daß die Formhohlräume (32) der Gießform (47) von Schalen (48) umgeben sind, die aus mindestens einem von nichtmetallischen Beimengungen freien Metall aus der Gruppe Tantal, Niob, Zirkonium und/oder deren Legierungen bestehen und die in einem Grundkörper (49) der Gießform (47) angeordnet sind, der aus mindestens einem nichtmetallischen Werkstoff wie Graphit und Siliziumnitrid besteht.

16. Gießform nach einem der Ansprüche 14 und 15, **dadurch gekennzeichnet,** daß die Schalen (48) auswechselbar in den Grundkörper (49) eingesetzt sind und ihre Dicke mindestens 2 mm beträgt.

17. Gießform nach Anspruch 16**, dadurch gekennzeichnet,** daß die Gießform (20, 47) aus mehreren, zur Rotationsachse (A-A) koaxialen Scheiben (30, 31) zusammengesetzt ist, in die die Schalen (48) mit einer Verbindung zum Eingußkanal (21) eingesetzt sind.

## Claims

1. Process to manufacture cast parts from a molten mass of a reactive metal of the group titanium, titanium alloys and titanium base alloys in a non-reactive atmosphere and in a re-usable casting mould (20, 47), configured as a spinning casting mould with axis of rotation (A-A) and a central sprue (21), which is connected with a number of die cavities (32) oriented away from the axis of rotation (A-A), characterised by limitation of the quenching speed by
a) Use of a casting mould (20, 47) which consists, at least on its surface coming into contact with the molten mass (32), of at least one metal free of non-metallic inclusions from the group tantalum, niobium, zirconium and/or alloys. thereof, and
b) Pre-heating of the casting mould (20, 47) before casting.

2. Process as per claim 1 characterised by use of a casting mould (20, 47) which consists of a tantalum base alloy, at least on its surface coming into contact with the molten mass.

3. Process as per claim 1 characterised by setting the preheating temperature below the liquidus temperature of the molten mass to be poured.

4. Process as per claim 3 characterised by setting the preheating temperature between 800°C and the solidus temperature of the molten mass to be poured.

5. Process as per claim 1 characterised by carrying out the casting of the molten mass into casting mould (20, 47) in a closed moulding chamber (5) at a pressure less than 100 mbar and preferably less than 10 mbar.

6. Process as per claim 1 characterised by introducing an inert gas, preferably at least one noble gas of the group argon and helium, into the moulding chamber (5) in order to increase the speed of cooling of the casting mould (20, 47) after casting.

7. Process as per claim 6 characterised by placing the inert gas in the moulding chamber (5) at a pressure between 100 mbar and atmospheric.

8. Casting mould to manufacture cast parts from a molten mass of a reactive metal from the group titanium, titanium alloys and titanium base alloys, configured as a reusable spinning casting mould with axis of rotation (A-A) and a central sprue (21), which is connected with a number of die cavities (32) oriented away from the axis of rotation (A-A)
characterised by the material of casting mould (20, 47) which consists, at least on its surface coming into contact with the molten mass (32a), of at least one metal free of non-metallic inclusions from the group tantalum, niobium, zirconium and/or alloys thereof.

9. Casting mould as per claim 8 characterised by the material of the mould, which consists of a tantalum base alloy, at least on its surface (32a) coming into contact with the molten mass.

10. Casting mould as per claim 9 characterised by the material of the mould containing at least 50% tantalum by weight.

11. Casting mould as per claim 8 characterised by the material of the mould containing at least one further metal from the group titanium, hafnium, tungsten and vanadium.

12. Casting mould as per claim 8 characterised by the material of the mould containing not only at least 50% tantalum by weight, but also at least one of the metals titanium, zirconium and tungsten.

13. Casting mould as per claim 12 characterised by the proportion of the metals titanium, zirconium and tungsten in total not exceeding 30% by weight.

14. Casting mould as per claim 11 characterised by the die cavities (32) of the casting mould (47) being surrounded by bowls consisting of at least one metal of the group tantalum, niobium, zirconium and/or alloys thereof, free of non-metallic inclusions, arranged in a base body (49) of the casting mould (47) consisting of at least one of the metals iron, nickel-titanium , titanium alumide and/or alloys thereof, preferably iron base alloys, nickel base alloys or austenitic heat-resistant steels, titanium, titanium alloys or titanium alumide.

15. Casting mould as per claim 11 characterised by the die cavities (32) of the casting mould (47) being surrounded by bowls (48) which consist of at least one metal of the group tantalum, niobium, zirconium and/or alloys thereof, free of non-metallic inclusions and arranged in a base body (49) of the casting mould (47) consisting of at least one non-metallic material such as graphite and silicon nitride.

16. Casting mould as per one of claims 14 and 15 characterised by bowls (48) at least 2 mm thick, removably inserted in base body (49).

17. Casting mould as per claim 16 characterised by the casting mould (20, 47) comprising several discs (30, 31) co-axial to the axis of rotation (A-A) into which the bowls (48) are inserted with a connection to the sprue (21).

## Revendications

1. Procédé pour la réalisation de pièces de fonderie à partir d'une fonte d'un métal réactif parmi le groupe comprenant le titane, les alliages de titane et les alliages à base de titane, dans une atmosphère non réactive et dans un moule de coulée (20, 47) réutilisable, réalisé sous forme d'un moule de coulée centrifuge et comprenant un axe de rotation (A - A) et un canal de coulée central (21), celui-ci étant en communication avec une pluralité de cavités de moulage (32) dirigées en éloignement de l'axe de rotation (A - A),
caractérisé en ce que la vitesse de trempe de la fonte est limitée par le fait que :
a) on utilise un moule de coulée (20, 47) qui est constitué, au moins au niveau de sa surface (32a) qui vient en contact avec la fonte, en au moins un métal, exempt d'additifs non métalliques, du groupe comprenant le tantale, niobium, zirconium et/ou leurs alliages, et
b) le moule de coulée (20, 47) est préchauffé avant l'opération de coulée.

2. Procédé selon la revendication 1, caractérisé en ce que l'on utilise un moule de coulée (20, 47) qui est constitué par un alliage à base de tantale, au moins au niveau de sa surface venant en contact avec la fonte.

3. Procédé selon la revendication 1, caractérisé en ce que la température de préchauffage est établie au-dessous de la température du liquidus de la fonte à couler.

4. Procédé selon la revendication 3, caractérisé en ce que la température de préchauffage est établie entre 800°C et la température du solidus de la fonte à couler.

5. Procédé selon la revendication 1, caractérisé en ce que l'opération de coulée de la fonte dans le moule de coulée (20), 47) est exécutée dans une chambre de moule fermée (5) sous une pression inférieure à 100 mbar, de préférence inférieure à 10 mbar.

6. Procédé selon la revendication 1, caractérisé en ce que, pour augmenter la vitesse de refroidissement du moule de coulée (20, 47) après l'opération de coulée, on introduit un gaz inerte, de préférence au moins un gaz rare du coupe formé par l'argon et l'hélium, dans la chambre de moulage (5).

7. Procédé selon la revendication 6, caractérisé en ce que le gaz inerte est introduit dans la chambre de moulage (5) sous une pression entre 100 mbar et la pression atmosphérique.

8. Moule de coulée pour la production de pièces de fonderie avec une fonte d'un métal réactif parmi le groupe formé par le titane, les alliages de titane et les alliages à base de titane, réalisé sous forme d'un moule centrifuge réutilisable et présentant un axe de rotation (A - A) et un canal d'entrée central (21), celui-ci communiquant avec une pluralité de cavités de moule (32), lesquelles sont dirigées en éloignement de l'axe de rotation (A - A),
caractérisé en ce que le matériau du moule de coulée (20, 47) est constitué, au moins au niveau des surfaces (32a) venant en contact avec la fonte, en au moins un métal exempt d'additifs non métalliques, choisi parmi le groupe formé par le tantale, niobium, zirconium et/ou leurs alliages.

9. Moule de coulée selon la revendication 8, caractérisé en ce que le matériau du moule, au moins au niveau de la surface (32a) venant en contact avec la fonte, est un alliage à base de tantale.

10. Moule de coulée selon la revendication 9, caractérisé en ce que le matériau du moule contient au moins 50% en poids de tantale.

11. Moule de coulée selon la revendication 8, caractérisé en ce que le matériau du moule contient encore au moins un autre métal du groupe constitué par le titane, hafnium, tungstène et vanadium.

12. Moule de coulée selon la revendication 8, caractérisé en ce que le matériau du moule contient, en plus d'au moins 50% en poids de tantale, l'un au moins des métaux parmi le titane, zirconium et tungstène.

13. Moule de coulée selon la revendication 12, caractérisé en ce que la proportion des métaux titane, zirconium et tungstène, s'élève au total au maximum à 30% en poids.

14. Moule de coulée selon la revendication 11, caractérisé en ce que les cavités de moulage (32) du moule (47) sont entourées par des coquilles (48), réalisées en au moins un métal, exempt d'additifs non métalliques, du groupe constitué par le tantale, niobium, zirconium et/ou leurs alliages, et agencées dans un corps de base (49) du moule de coulée (47), ledit corps de base étant réalisé en l'un au moins des métaux que sont le fer, le nickel, le titane, le titane alumineux et/ou leurs alliages, de préférence des alliages à base de fer, des alliages à base de nickel, ou bien des aciers réfractaires austénitiques, du titane, des alliages de titane, ou du titane alumineux.

15. Moule de coulée selon la revendication 11, caractérisé, en ce que les cavités de moulage (32) du moule (47) sont entourées par des coquilles (48), réalisées en au moins un métal, exempt d'additifs non métalliques, du groupe constitué par le tantale, niobium, zirconium et/ou leurs alliages, et agencées dans un corps de base (49) du moule de coulée (47), ledit corps de base étant constitué en au moins un matériau non métallique tel que du graphite et du nitrure de silicium.

16. Moule de coulée selon l'une des revendications 14 et 15, caractérisé en ce que les coquilles (48) sont mises en place de manière interchangeable dans le corps de base (49), et en ce que leur épaisseur s'élève à au moins 2 mm.

17. Moule de coulée selon la revendication 16, caractérisé en ce que le moule de coulée (20, 47) est composé de plusieurs disques (30, 31) coaxiaux vis-à-vis de l'axe de rotation (A - A), dans lesquels les coquilles (48) sont mises en place avec une liaison vers le canal d'entrée (21).
